# EUROPEAN PATENT APPLICATION

(11) **EP 4 257 693 A1**
(43) Date of publication of application: **11.10.2023**
(21) Application number: 21903685.2
(22) Date of filing: 23.11.2021
(51) Int. Cl.: C12N 15/81, C12N 9/02, C12N 9/80, C12N 9/16, C12N 9/12, C12P 13/00, C12P 9/00

(54) **SACCHAROMYCES CEREVISIAE MUTANT STRAINS FOR PRODUCING SPHINGOSINE AND SPHINGOSINE-1-PHOSPHATE AND METHODS FOR PRODUCING SAME**

(30) Priority: 07.12.2020 KR 20200169892
(71) Applicant: Korea Institute of Industrial Technology, Cheonan-si, Chungcheongnam-do 31056 (KR)
(72) Inventor: YU, Byung Jo, Suwon-si Gyeonggi-do 16582 (KR); LEE, Sung Jin, Incheon 22711 (KR); JANG, Ji Yeon, Cheonan-si Chungcheongnam-do 31120 (KR); JANG, In Seung, Cheonan-si Chungcheongnam-do 31037 (KR)
(74) Representative: Schön, Christoph
(86) International application number: PCT/KR2021/017220
(87) International publication number: WO 2022/124639

(57) **Abstract**

Provided are: a saccharomyces cerevisiae mutant strain for producing sphingosine; a saccharomyces cerevisiae mutant strain for producing sphingosine-1-phosphate; methods for producing same; and a method of producing sphingosine or sphingosine-1-phosphate by using the strains. Particularly, provided is a Saccharomyces cerevisiae mutant strain for producing sphingosine, into which a human sphingolipid desaturase 1 (hDES1) gene and an alkaline ceramidase 1 (ACER1) gene are introduced.

## Description

### [Technical Field]

The present invention relates to a *Saccharomyces cerevisiae* mutant strain for producing sphingosine, a *Saccharomyces cerevisiae* mutant strain for producing sphingosine 1-phosphate, methods for preparing the same, and a method of producing sphingosine or sphingosine 1-phosphate using the same.
National research and development project that supported this invention
Department Name: Ministry of Science and ICT
Assignment identification number: 1711100950
Assignment number: E0190054
Name of project management institution: KOREA INSTITUTE OF INDUSTRIAL TECHNOLOGY

### [Background Art]

Sphingosine, which is represented by (2S,3R)-2-aminooctadec-4-trans-ene-1,3-diol, is a C18 amino alcohol containing an unsaturated hydrocarbon chain. Sphingosine constitutes the major part of sphingolipids. Sphingosine is a precursor to ceramide, which is an ingredient of the skin layer, and is known to have moisturizing, soothing and anti-aging effects.

In addition, sphingosine 1-phosphate (S1P) may be produced by phosphorylation of sphingosine. Sphingosine 1-phosphate is a signal transmitter and acts as an essential factor that mediates collagen activity, blood vessel regeneration, and immune cell activity and has an anticancer effect.

Such sphingosine and sphingosine 1-phosphate have potential for use as raw materials for cosmetics and pharmaceuticals. A known method of producing sphingosines and ceramides is chemical synthesis using plant-derived extracts. However, the chemical synthesis has low chemical conversion efficiency and very low production concentration.

Another known method of producing sphingosines and ceramides is simple fermentation in wild strains. For example, Patent Document 1 discloses a method for mass-producing long chain bases of sphingolipids, specifically sphingolipids such as sphingosine and phytosphingosine, which are key precursors for the synthesis of ceramides or ceramide derivatives, based on optimization of fermentation conditions of *Pichia ciferrii* DSCC 7-25, which is an excellent sphingolipid-producing strain, and methods of isolating and purifying sphingolipids. However, such a simple fermentation in wild strains also has a problem of difficulty in commercialization due to low fermentation production concentration.

Meanwhile, sphingosine 1-phosphate is prepared by a chemical method using sphingosine as a precursor, but yield and production thereof are too low for industrialization and a fermentation-based production method using microorganisms has not been established to date. For this reason, it is difficult to use sphingosine 1-phosphate for cosmetics or pharmaceuticals due to the high price thereof despite the excellent bioactivity thereof.

Therefore, there is demand for development of strains capable of producing sphingosine and sphingosine 1-phosphate with high efficiency using metabolic engineering based on safe yeast strains.

### [Disclosure]

### [Technical Problem]

Therefore, it is one object (aspect) of the present invention to provide a *Saccharomyces cerevisiae* mutant strain for producing sphingosine.

It is another object (aspect) of the present invention to provide a *Saccharomyces cerevisiae* mutant strain for producing sphingosine 1-phosphate.

It is another object (aspect) of the present invention to provide a method for preparing the *Saccharomyces cerevisiae* mutant strain for producing sphingosine.

It is another object (aspect) of the present invention to provide a method for preparing the *Saccharomyces cerevisiae* mutant strain for producing sphingosine 1-phosphate.

It is another object (aspect) of the present invention to provide a method of producing sphingosine including culturing the strain in a medium.

It is another object (aspect) of the present invention to provide a method of producing sphingosine 1-phosphate including culturing the strain in a medium.

The objects (aspect) of the present invention are not limited to those described above. Other objects of the present invention will be clearly understood from the following description.

### [Technical Solution]

Advantages, features, and methods to accomplish the same according to the present invention will be clearly understood from the following preferred embodiments with reference to the attached drawings. However, the present invention is not limited to the embodiments and may be embodied in different forms. The embodiments are suggested only to completely inform those skilled in the art of the scope of the present invention. The present invention will be defined only by the claims. In other words, a variety of modifications to the embodiments of the present invention are possible. The following embodiments of the present invention should not be construed as limiting the scope of the present invention, and those skilled in the art will appreciate that all modifications, additions and substitutions are possible.

All of the terms (including technical and scientific terms) used herein may be understood as having meanings that can be commonly understood by those skilled in the art to which the present invention pertains. In addition, terms defined in commonly used dictionaries should not be interpreted ideally or excessively unless explicitly specifically defined.

The term "and/or" used herein includes a designated single item and any combination of one or more items. Singular forms are intended to include plural forms as well, unless the context clearly indicates otherwise. It will be further understood that the terms "comprises" and/or "comprising" used herein do not preclude the presence or addition of at least one element other than the mentioned element. Numerical ranges indicated using the term "to" indicate numerical ranges including the values set forth before and after the term as lower and upper limits, respectively. The term "about" or "approximately" means a value or numerical range within 20% of the value or numerical range set forth after the term.

As used herein, the term "enhancement of activity", "increase in activity" or "increased activity" of an enzyme, polypeptide or protein may mean that the enzyme, polypeptide or protein exhibits activity increased to a sufficient degree, and means that cells or isolated polypeptides exhibit a high level of activity compared to the level of activity measured in comparable cells of the same species or native polypeptide thereof. That is, the activity of the polypeptide is increased by about 5.0% or more, about 10% or more, about 15% or more, about 20% or more, about 30% or more, about 50% or more, about 60% or more, about 70% or more, or about 100% or more, compared to the same biochemical activity of an original unmanipulated polypeptide.

An increase in the activity of a polypeptide may be obtained by an increase in the expression or specific activity of the polypeptide. The increase in expression may be due to introduction of a polynucleotide encoding a polypeptide into cells, an increase in copy number in cells, or mutation of a regulatory region of the polynucleotide. Polynucleotides introduced exogenously or having an increased copy number may be endogenous or exogenous. The endogenous gene refers to a gene present in genetic material contained in the microorganism. The exogenous gene refers to a gene introduced into a host cell, such as a gene integrated into the genome of the host cell, and the introduced gene may be homologous or heterologous to the host cell into which it is introduced.

As used herein, the term "increase in copy number" may be due to the integration or amplification of genes and includes a case where genes that do not exist in unmanipulated cells are imparted to the cells by genetic manipulation. The integration of genes may be performed via a vehicle such as a vector. The "introduction" may be transient introduction, in which the gene is not integrated into the genome, or an insertion of the gene into the genome. The introduction may be performed, for example, by introducing, into the cell, a vector, into which a polynucleotide encoding a desired polypeptide is inserted, and then replicating the vector in the cell or integrating the polynucleotide into the genome.

The vector used herein is not particularly limited as long as it can be replicated in a host cell and any vector known in the art can be used. Examples of commonly used vectors include natural or recombinant plasmids, cosmids, viruses and bacteriophages. Examples of phage vectors or cosmid vectors include pWE15, M13, MBL3, MBL4, IXII, ASHII, APII, t10, t11, Charon4A, Charon21A and the like. Examples of plasmid vectors include pBR, pUC, pBlueScript II, pGEM, pTZ, pCL, and pET plasmid vectors. Specifically, these plasmid vectors include, but are not limited to, pDZ, pACYC177, pACYC184, pCL, pECCG117, pUC19, pBR322, pMW118, pCC1BAC, pSKH, pRS-413, pRS-414, pRS-415, pBCA, and pYLI vectors.

The term "gene" refers to a nucleic acid fragment that expresses a specific protein and includes a coding region or a regulatory region(sequence), such as a 5'-non-coding sequence or a 3'-non-coding sequence, outside the coding region. The regulatory region may include a promoter, an enhancer, an operator, a ribosome binding site, a polyA binding sequence, a terminator region, and the like.

The term "transformation" means introducing a vector containing a polynucleotide encoding a target protein into a host cell so that the protein encoded by the polynucleotide can be expressed in the host cell. The transformed polynucleotide may be inserted into the chromosome of the host cell and/or located outside the chromosome as long as it can be expressed in the host cell. In addition, the polynucleotide includes DNA and RNA encoding a target protein. The polynucleotide may be introduced in any mode as long as it can be introduced into a host cell and expressed. For example, the polynucleotide may be introduced into a host cell in the form of an expression cassette which is a genetic construct containing all elements required for self-expression. The expression cassette may include a promoter, a transcription termination signal, a ribosome-binding site, and a translation termination signal, each of which is operably linked to the polynucleotide. The expression cassette may be in the form of an expression vector capable of self-replication. In addition, the polynucleotide may be introduced as its own original form into a host cell and operably linked to a sequence necessary for expression in the host cell, but is not limited thereto. The transformation includes any method of introducing a nucleic acid into a cell and may be performed by selecting an appropriate standard technique known in the art depending on the host cell. Examples of the transformation method include, but are not limited to, electroporation, calcium phosphate (CaPO₄) precipitation, calcium chloride (CaCl₂) precipitation, microinjection, polyethylene glycol (PEG) method, EAE-dextran method, cationic liposomal method, and lithium acetate-DMSO method, and the like.

The term "operably linked" means that a polynucleotide sequence is functionally linked to a promoter sequence that initiates and mediates transcription of a polynucleotide encoding the target protein of the present application. The operable linkage may be prepared using genetic recombination techniques known in the art and site-specific DNA cleavage and linkage may be performed using cutting and linking enzymes in the art, without being limited thereto.

The term "heterologous" means foreign, rather than native.

The term "secretion" means the movement of a substance from inside cells into the periplasmic space or the extracellular environment.

The terms "cell", "strain", are "microorganism" are used interchangeably and include bacteria, yeasts, molds, and the like.

A "decrease in activity" or "decreased activity" of an enzyme or polypeptide means that the cell or isolated enzyme or polypeptide exhibits lower or no activity compared to the activity measured in comparable cells of the same species or its native polypeptide. That is, the activity of the polypeptide is decreased by about 10% or more, about 20% or more, about 30% or more, about 40% or more, about 50% or more, about 55% or more, about 60% or more, about 70% or more, about 75% or more, about 80% or more, about 85% or more, about 90% or more, about 95% or more, or about 100%, compared to the biochemical activity of the unmanipulated wild-type polypeptide. The decrease in enzymatic activity includes when the enzymatic activity is absent or reduced although the enzyme is expressed, or when a gene encoding the enzyme is not expressed, or when the expression level thereof is reduced compared to an unmanipulated wild-type polypeptide.

The decrease in enzymatic activity may be caused by deletion or disruption of a gene encoding the enzyme. The term "deletion" or "disruption" of a gene means mutation, substitution, or deletion of a part or all of a gene or a part or all a regulatory factor such as a promoter thereof, or a terminator region thereof, or insertion of at least one base into the gene so that the gene is not expressed, the expression level thereof is reduced or the enzyme activity is absent or reduced even if expressed. The deletion or disruption of the gene may be achieved by genetic manipulation such as homologous recombination, mutagenesis, or molecular evolution. Where cells contain a plurality of identical genes or contain two or more different polypeptide paralogs, one or more of the genes may be deleted or disrupted.

The term "sequence identity" of nucleic acids or polypeptides refers to the degree of identity of bases or amino acid residues between sequences after aligning both sequences with each other to maximize matching in a specific region for comparison. Sequence identity is measured by optimally aligning and comparing two sequences in a specific region for comparison and a part of the sequence region for comparison may be added or deleted compared to a reference sequence.

The enzymes in the present invention may include enzymes having a homology of 80% or more, 85% or more, 90% or more, 91% or more, 92% or more, 93% or more, 94% or more, 95% or more, 96% or more, 97% or more, 98% or more, or 99% or more of the enzymes described as long as they have the same or corresponding biological activity as each enzyme.

Percentage sequence identity can be calculated by, for example, comparing two optimally aligned sequences over the entire region for comparison, determining the number of positions in which identical amino acids or nucleic acids occur in both sequences to obtain the number of matched positions, dividing the number of matched positions by the total number of positions within the comparison range (i.e., range size) , and multiplying the result by 100 to obtain a percentage of sequence identity.

The percent of sequence identity is obtained using a known sequence comparison standard program for calculating parameters such as score, identity and similarity, or comparing sequences by hybridization experiments performed under defined stringent conditions. Examples of the program include BLASTN (NCBI), CLC Main Workbench (CLC bio), MegAlign^{™} (DNASTAR Inc) and the like. The appropriate hybridization conditions defined fall within the scope of the corresponding art and may be determined by methods well known to those skilled in the art (for example, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York). As used herein, the term "stringent conditions" means conditions that allow for specific hybridization between polynucleotides. For example, such conditions are described in detail in the literature (for example, J. Sambrook et al., the same as above).

Different levels of sequence identity may be used to identify polypeptides or polynucleotides having identical or similar functions or activity in different species. For example, sequence identity of 50% or more, 55% or more, 60% or more, 65% or more, 70% or more, 75% or more, 80% or more, 85% or more, 90% or more, 95% or more, 96% or more, 97% or more, 98% or more, 99% or more, or 100%, or the like may be used.

"Sphingosine" is represented by (2*S*,3*R*)-2-aminooctadec-4-trans-ene-1,3-diol. Sphingosine is an 18C carbon amino alcohol containing an unsaturated hydrocarbon chain and constitutes the major part of sphingolipids.

"Sphingosine 1-phosphate", "sphingosine 1-P", "S1P", and "D-erythro-sphingosine 1-phosphate" are used interchangeably and are produced through phosphorylation of sphingosine.

The present inventors introduced a gene group capable of biosynthesizing sphingosine and sphingosine 1-phosphate in a safe yeast strain, *Saccharomyces cerevisiae.* In addition, the production concentration and productivity of sphingosine and sphingosine 1-phosphate were improved by removing competing genes and enhancing the expression of precursor-related biosynthetic genes.

One embodiment (or one aspect) of the present invention to solve the above problems provides a *Saccharomyces cerevisiae* mutant strain for producing sphingosine into which a hDES1 (human sphingolipid desaturase 1) gene and an ACER1 (alkaline ceramidase 1) gene are introduced.

hDES1 (human sphingolipid desaturase 1) is a human-derived gene. A polynucleotide sequence encoding the hDES1 gene may be represented by SEQ ID NO: 1, but is not limited thereto.

ACER1 (alkaline ceramidase 1) may be a human- or mouse-derived gene. ACER1 may be a human-derived gene. The human-derived ACER1 is hACER1. A polynucleotide sequence encoding the hACER1 gene may be represented by SEQ ID NO: 2, but is not limited thereto.

The mutant strain may be a mutant strain having a sphingolipid metabolic pathway redesigned using a wild-type strain of *Saccharomyces cerevisiae* as a parent strain. Any strain may be used as the parent strain of *Saccharomyces cerevisiae* without limitation as to the type thereof as long as it has a sphingolipid metabolic pathway.

The strain may be genetically engineered to improve the expression of the hDES1 gene and the ACER1 gene compared to a non-genetically engineered strain. Since the hDES1 gene and the ACER1 gene do not exist in the wild-type strain, the hDES1 gene and the ACER1 gene may be introduced into the strain by genetic manipulation so that they are expressed or overexpressed therein. The non-genetically engineered strain refers to a wild strain or a parental cell from which the strain is derived.

The expression or overexpression of the hDES1 gene and the ACER1 gene may be achieved by various methods known to those skilled in the art. For example, expression may be improved by increasing the number of gene copies or using a regulatory substance such as an inducer or a repressor. The increase in the number of gene copies may be due to introduction or amplification of the gene. That is, the increase in the number of gene copies may be achieved by introducing a vector, an expression cassette, or the like containing an operably linked regulatory factor and the gene into a host cell.

Alternatively, the increase in hDES1 and ACER1 enzymatic activity may be due to modification of regulatory sequence of the gene expression. The regulatory sequence may be a promoter sequence or a transcription terminator sequence for expression of the gene. In addition, the regulatory sequence may be a sequence encoding a motif capable of influencing gene expression. The motif may be, for example, a secondary structure-stabilizing motif, an RNA-destabilizing motif, a splice-activating motif, a polyadenylation motif, an adenine-rich sequence, or an endonuclease recognition site.

A sphingomyelin phosphodiesterase 1 (SMPD1) gene may be further introduced into the strain. SMPD1 is also referred to as "SMase". SMPD1 may be a human-derived gene. The human-derived SMPD1 is hSMPD1. A polynucleotide sequence encoding the hSMPD1 gene may be represented by SEQ ID NO: 3, but is not limited thereto. The SMPD1 gene may be involved in a pathway that converts inositol-phosphoceramide (IPC), mannose-inositol-phosphoceramide (MIPC) or mannose-(inositol-P)₂phosphoceramide (M(IP)2C) to ceramide, a precursor of sphingosine.

The strain may have enhanced synthesis of DHS (dihydrosphingosine). The enhanced synthesis of DHS means that the metabolic pathway of the yeast strain focuses on the synthesis of DHS compared to the wild-type strain in the sphingolipid metabolic pathway, so that the protein is produced at a high level. For this purpose, the sphingolipid metabolic pathway that generally occurs in the wild strain may be changed. The change of the metabolic pathway may be elimination of competing genes and/or enhancement of protein biosynthetic gene expression.

The strain may have reduced activity of at least one enzyme involved in a pathway for degrading DHS (dihydrosphingosine) or converting DHS into other products. The strain may be in a state in which a gene encoding at least one enzyme involved in a pathway for degrading DHS (dihydrosphingosine) or converting DHS into other products is deleted or disrupted. For example, the strain may have reduced activity of at least one enzyme involved in a pathway for converting DHS (dihydrosphingosine) into DHSP (dihydrosphingosine 1-phosphate, DHS-1-P). Alternatively, the strain may have reduced activity of at least one enzyme involved in a pathway for converting PHS into PHSP (phytosphingosine 1-phosphate, PHS-1-P). Alternatively, the strain may have reduced activity of at least one enzyme involved in a pathway for converting DHSP and/or PHSP into ethanolamine and fatty aldehyde.

The strain may have enhanced synthesis of DHC (dihydroceramide). The enhanced synthesis of DHC means that the metabolic pathway of the yeast strain focuses on the synthesis of DHC compared to the wild-type strain in the sphingolipid metabolic pathway, so that the protein is produced at a high level. For this purpose, the sphingolipid metabolic pathway that generally occurs in the wild strain can be changed. The change of the metabolic pathway may be deletion of competing genes and/or enhancement of protein biosynthetic gene expression.

The strain may have reduced activity of at least one enzyme involved in a pathway for degrading dihydroceramide (DHC) or converting DHC into other products. The strain may be in a state in which a gene encoding at least one enzyme involved in a pathway for degrading dihydroceramide (DHC) or converting DHC into other products is deleted or disrupted. For example, the strain may have reduced activity of at least one enzyme involved in a pathway for converting DHC into phytoceramide (PHC).

The strain may have reduced activity of at least one enzyme involved in a pathway for converting DHS into DHSP. Alternatively, the enzyme involved in the pathway for converting DHS into DHSP may be sphingoid long chain base kinase 4 (LCB4), sphingoid long chain base kinase 5 (LCB5), or a combination thereof.

The strain may have reduced activity of at least one enzyme involved in a pathway for converting PHS into PHSP. The enzyme involved in the pathway for converting PHS into PHSP may be LCB4, LCB5, or a combination thereof.

The strain may have reduced activity of at least one enzyme involved in a pathway for converting DHSP and/or PHSP into ethanolamine and fatty aldehyde. The enzyme involved in a pathway for converting DHSP and/or PHSP into ethanolamine and fatty aldehyde may be DPL1 (dihydrosphingosine phosphate lyase) .

The strain may have reduced activity of at least one enzyme involved in a pathway for converting DHS into PHS. The enzyme involved in a pathway for converting DHS into PHS may be SUR2 (sphinganine C4-hydroxylase) .

The strain may have reduced activity of at least one enzyme involved in a pathway for converting DHC into PHC. The enzyme involved in a pathway for converting DHC into PHC may be SUR2.

Therefore, the strain may be in a state in which at least one gene of sphingoid long chain base kinase 4 (LCB4), sphingoid long chain base kinase 5 (LCB5), dihydrosphingosine phosphate lyase (DPL1), and sphinganine C4-hydroxylase (SUR2) has been deleted or disrupted.

The strain may have improved activity of an enzyme that catalyzes a reaction of converting palmitoyl-CoA into 3-ketodihydrosphingosine. The strain may be in a state in which a gene encoding the enzyme that catalyzes the reaction of converting palmitoyl-CoA to 3-ketodihydrosphingosine is expressed at a higher level than a non-genetically engineered strain. The expression level may be mRNA- or protein-level expression. The expression level may be increased by about 5% or more, about 10% or more, about 15% or more, about 20% or more, about 30% or more, about 50% or more, about 60% or more, about 70% or more, about 100% or more, 200% or more, or 300% or more.

In an exemplary embodiment, the strain may overexpress at least one gene selected from serine palmitoyltransferase 1 (LCB1), serine palmitoyltransferase 2 (LCB2) and serine palmitoyltransferase-regulating protein (TSC3). The overexpression may be achieved by introducing a gene.

The strain may have improved activity of an enzyme that catalyzes a reaction of converting 3-ketodihydrosphingosine into DHS. The strain may be a state in which a gene encoding an enzyme that catalyzes a reaction of converting 3-ketodihydrosphingosine into DHS is expressed at a higher level than a non-genetically engineered strain. The expression level may be mRNA- or protein-level expression. The expression level may be increased by about 5% or more, about 10% or more, about 15% or more, about 20% or more, about 30% or more, about 50% or more, about 60% or more, about 70% or more, about 100% or more, 200% or more, or 300% or more.

In an exemplary embodiment, the strain may overexpress a 3-ketodihydrosphingosine reductase (TSC10) gene. The overexpression may be achieved by introducing a gene.

The strain may have improved activity of an enzyme that catalyzes a reaction of converting DHS into DHC. The strain may be a state in which a gene encoding an enzyme that catalyzes a reaction of converting DHS into DHC is expressed at a higher level than a non-genetically engineered strain. The expression level may be mRNA- or protein-level expression. The expression level may be increased by about 5% or more, about 10% or more, about 15% or more, about 20% or more, about 30% or more, about 50% or more, about 60% or more, about 70% or more, about 100% or more, 200% or more, or 300% or more.

In an exemplary embodiment, the strain may overexpress at least one gene selected from sphingosine N-acyltransferase (LAG1), sphingosine N-acyltransferase (LAC1), and ceramide synthase subunit (LIP1). The overexpression may be achieved by introducing a gene.

In another aspect, the present invention provides a *Saccharomyces cerevisiae* mutant strain for producing sphingosine 1-phosphate, constructed by introducing a hSPHK1 (human sphingosine kinase 1) gene into the *Saccharomyces cerevisiae* mutant strain for producing sphingosine.

Therefore, the *Saccharomyces cerevisiae* mutant strain for producing sphingosine 1-phosphate may include genetic manipulations of the *Saccharomyces cerevisiae* mutant strain for producing sphingosine.

In an exemplary embodiment, the *Saccharomyces cerevisiae* mutant strain for producing sphingosine 1-phosphate may include all genetic manipulations of the *Saccharomyces cerevisiae* mutant strain for producing sphingosine, except that the hSPHK1 gene rather than the ACER1 gene is introduced.

In an exemplary embodiment, the ACER1 gene may be optionally introduced.

Th hSPHK1 (human sphingosine kinase 1) is a human-derived gene. The polynucleotide sequence encoding the hSPHK1 gene may be represented by SEQ ID NO: 4, but is not limited thereto.

The strain may be genetically engineered to increase the expression of the hSPHK1 gene compared to a non-genetically engineered strain. Since the hSPHK1 gene does not exist in the wild-type strain, the hSPHK1 gene may be introduced into the strain by genetic manipulation so that it can be expressed or overexpressed. The non-genetically engineered strain refers to a wild strain or a parental cell from which the strain is derived.

The expression or overexpression of the hSPHK1 gene may be achieved by various methods known to those skilled in the art. For example, expression may be improved by increasing the number of gene copies or using a regulatory substance such as an inducer or a repressor. The increase in the number of gene copies may be due to introduction or amplification of the gene. That is, the increase in the number of gene copies may be achieved by introducing a vector, an expression cassette, or the like containing an operably linked regulatory factor and the gene into a host cell.

Alternatively, the increase in hSPHK1 enzymatic activity may be due to modification of expression regulatory sequences of the gene. The regulatory sequence may be a promoter sequence or a transcription terminator sequence for expression of the gene. In addition, the regulatory sequence may be a sequence encoding a motif capable of influencing gene expression. The motif may be, for example, a secondary structure-stabilizing motif, an RNA-destabilizing motif, a splice-activating motif, a polyadenylation motif, an adenine-rich sequence, or an endonuclease recognition site.

Sphingosine may be converted into sphingosine 1-phosphate by human sphingosine kinase 1 (hSPHK1).

In another aspect, the present invention provides a method of producing the *Saccharomyces cerevisiae* mutant strain for producing sphingosine, including:
(1) introducing a hDES1 (human sphingolipid desaturase 1) gene and an ACER1 (alkaline ceramidase 1) gene into a *Saccharomyces cerevisiae* strain.

The method may further include at least one of the following steps:
(2) introducing a SMPD1 (sphingomyelin phosphodiesterase 1) gene into the *Saccharomyces cerevisiae* strain;
(3) deleting at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase) from the *Saccharomyces cerevisiae* strain; and
(4) overexpressing at least one gene of LCB1 (serine palmitoyltransferase 1), LCB2 (serine palmitoyltransferase 2), TSC3 (serine palmitoyltransferase-regulating protein TSC3), TSC10 (3-ketodihydrosphingosine reductase), LAG1 (sphingosine N-acyltransferase), LAC1 (sphingosine N-acyltransferase), and LIP1 (ceramide synthase subunit) in the *Saccharomyces cerevisiae* strain.

In another aspect, the present invention provides a method of producing the *Saccharomyces cerevisiae* mutant strain for producing sphingosine 1-phosphate, including:
(1) introducing a hDES1(human sphingolipid desaturase 1) gene, an ACER1 (alkaline ceramidase 1) gene, and a hSPHK1 (human sphingosine kinase 1) gene into a *Saccharomyces cerevisiae* strain.

The method may further include at least one of the following steps:
(2)introducing a SMPD1 (sphingomyelin phosphodiesterase 1) gene into the *Saccharomyces cerevisiae* strain;
(3) deleting at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase) from the *Saccharomyces cerevisiae* strain; and
(4) overexpressing at least one gene of LCB1 (serine palmitoyltransferase 1), LCB2 (serine palmitoyltransferase 2), TSC3 (serine palmitoyltransferase-regulating protein TSC3), TSC10 (3-ketodihydrosphingosine reductase), LAG1 (sphingosine N-acyltransferase), LAC1 (sphingosine N-acyltransferase), and LIP1 (ceramide synthase subunit) in the *Saccharomyces cerevisiae.*

In another aspect, the present invention provides a method of producing the *Saccharomyces cerevisiae* mutant strain for producing sphingosine 1-phosphate, including:
(1) introducing a hDES1(human sphingolipid desaturase 1) gene, and a hSPHK1 (human sphingosine kinase 1) gene into a *Saccharomyces cerevisiae* strain.

The method may further include at least one of the following steps:
(2) introducing an ACER1 (alkaline ceramidase 1) gene into the *Saccharomyces cerevisiae* strain;
(3)introducing a SMPD1 (sphingomyelin phosphodiesterase 1) gene into the *Saccharomyces cerevisiae* strain;
(4) deleting at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase) from the *Saccharomyces cerevisiae* strain; and
(5) overexpressing at least one gene of LCB1 (serine palmitoyltransferase 1), LCB2 (serine palmitoyltransferase 2), TSC3 (serine palmitoyltransferase-regulating protein TSC3), TSC10 (3-ketodihydrosphingosine reductase), LAG1 (sphingosine N-acyltransferase), LAC1 (sphingosine N-acyltransferase), and LIP1 (ceramide synthase subunit) in the *Saccharomyces cerevisiae.*

In another aspect, the present invention provides a method of producing sphingosine including culturing the *Saccharomyces cerevisiae* mutant strain for producing sphingosine in a medium. As used herein, the term "culture" refers to growing microorganisms under appropriately controlled environmental conditions.

The culture may be performed in an appropriate medium under appropriate culture conditions known in the art. Those skilled in the art can easily control and use the medium and culture conditions depending on the type of strain. The culture method may include batch, continuous, or fed-batch culture, or a combination thereof. The strain may secrete sphingosine to the outside of the cell.

The medium may contain any one of various carbon sources, nitrogen sources, and trace element components.

The carbon source may, for example, include carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, organic acids such as acetic acid, or combinations thereof. The culture may be performed using glucose as a carbon source. The nitrogen source may include an organic nitrogen source such as peptone, a yeast extract, gravy, a malt extract, corn steep liquor (CSL), or soybean wheat, an inorganic nitrogen source such as urea, ammonium sulfate, ammonium chloride, ammonium phosphate, ammonium carbonate or ammonium nitrate, or a combination thereof. The medium may contain, as a phosphorus source, for example, potassium dihydrogen phosphate, dipotassium hydrogen phosphate and a sodium-containing salt thereof, or a metal salt such as magnesium sulfate or iron sulfate. The medium may contain amino acids, vitamins, and appropriate precursors. The medium or each component thereof may be added to the culture solution in a batch, fed-batch or continuous manner.

In addition, the pH may not be adjusted during culture, or the pH of the culture medium may be adjusted by adding a compound such as ammonium hydroxide, potassium hydroxide, ammonia, phosphoric acid, or sulfuric acid to a microbial culture medium in an appropriate manner. In addition, the formation of bubbles may be suppressed using an antifoaming agent such as fatty acid polyglycol ester during culture.

The culture may be performed under aerobic, microaerobic or anaerobic conditions. The term "aerobic condition" means a condition in which oxygen is sufficiently dissolved in the culture medium. The term "microaerobic condition" means a condition in which oxygen is supplied to the culture medium in a state in which air having a lower amount of oxygen than a normal atmospheric condition is in contact with the culture medium. The microaerobic condition may be created, for example, by supplying carbon dioxide or nitrogen to atmospheric air at a flow rate of about 0.1 to 0.4 vvm, about 0.2 to 0.3 vvm, or about 0.25 vvm. Further, the microaerobic condition may be created at an aeration rate of about 0 to 0.4 vvm, about 0.1 to 0.3 vvm, or about 0.15 to 0.25 vvm. The term "anaerobic condition" means a condition in which oxygen is not supplied to the culture medium. In one embodiment, the culture may be performed under an anaerobic or microaerobic condition.

The method may further include recovering sphingosine from the culture product. The recovering may be performed by isolating sphingosine from cells, a culture solution excluding the cells or both. The isolation of sphingosine from the culture product may be performed using separation and purification methods known in the art. The recovering may be achieved by centrifugation, chromatography, extraction, filtration, precipitation, or a combination thereof.

In another aspect, the present invention provides a method of producing sphingosine 1-phosphate including culturing the *Saccharomyces cerevisiae* mutant strain for producing sphingosine 1-phosphate in a medium.

The culture may be performed in an appropriate medium under appropriate culture conditions known in the art. Those skilled in the art can easily control and use the medium and culture conditions depending on the type of strain. The culture method may include batch, continuous, or fed-batch culture, or a combination thereof. The strain may secrete sphingosine to the outside of the cell.

The medium may contain any one of various carbon sources, nitrogen sources, and trace element components.

The carbon source may, for example, include carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, organic acids such as acetic acid, or combinations thereof. The culture may be performed using glucose as a carbon source. The nitrogen source may include an organic nitrogen source such as peptone, yeast extract, gravy, a malt extract, corn steep liquor (CSL), and soybean wheat, an inorganic nitrogen source such as urea, ammonium sulfate, ammonium chloride, ammonium phosphate, ammonium carbonate and ammonium nitrate, or a combination thereof. The medium may contain, as a phosphorus source, for example, potassium dihydrogen phosphate, dipotassium hydrogen phosphate and a sodium-containing salt thereof, or a metal salt such as magnesium sulfate or iron sulfate. The medium may contain amino acids, vitamins, and appropriate precursors. The medium or each component thereof may be added to the culture solution in a batch, fed-batch or continuous manner.

In addition, the pH may not be adjusted during culture, or the pH of the culture medium may be adjusted by adding a compound such as ammonium hydroxide, potassium hydroxide, ammonia, phosphoric acid, or sulfuric acid to a microbial culture medium in an appropriate manner. In addition, the formation of bubbles may be suppressed using an antifoaming agent such as fatty acid polyglycol ester during culture.

The culture may be performed under aerobic, microaerobic or anaerobic conditions. The term "aerobic condition" means a condition in which oxygen is sufficiently dissolved in the culture medium. The term "microaerobic condition" means a condition in which oxygen is supplied to the culture medium in a state in which air having a lower amount of oxygen than a normal atmospheric condition is in contact with the culture medium. The microaerobic condition may be created, for example, by supplying carbon dioxide or nitrogen to atmospheric air at a flow rate of about 0.1 to 0.4 vvm, about 0.2 to 0.3 vvm, or about 0.25 vvm. Further, the microaerobic condition may be created at an aeration rate of about 0 to 0.4 vvm, about 0.1 to 0.3 vvm, or about 0.15 to 0.25 vvm. The term "anaerobic condition" means a condition in which oxygen is not supplied to the culture medium. In one embodiment, the culture may be performed under anaerobic or microaerobic conditions.

The method may further include recovering sphingosine 1-phosphate from the culture product. The recovering may be performed by isolating sphingosine from cells, a culture solution excluding the cells or both. The isolation of sphingosine from the culture product may be performed using separation and purification methods known in the art. The recovering may be achieved by centrifugation, chromatography, extraction, filtration, precipitation, or a combination thereof.

### [Advantageous effects]

The *Saccharomyces cerevisiae* mutant strains according to embodiments of the present invention have an increased ability to produce sphingosine.

In addition, the *Saccharomyces cerevisiae* mutant strains according to embodiments of the present invention have the ability to produce sphingosine 1-phosphate.

The methods of producing sphingosine or sphingosine 1-phosphate according to embodiments of the present invention enable efficient production of sphingosine or sphingosine 1-phosphate. In addition, the produced sphingosine and sphingosine 1-phosphate are utilized in a variety of applications as raw materials for cosmetics and pharmaceuticals.

The effects according to the embodiments of the present invention are not limited to those exemplified above and other various effects are incorporated in the present specification.

### [Description of Drawings]

FIG. 1 is a codon-optimized polynucleotide sequence encoding hDES1;
FIG. 2 is a codon-optimized polynucleotide sequence encoding hACER1;
FIG. 3 is a codon-optimized polynucleotide sequence encoding hSMPD1;
FIG. 4 is a codon-optimized polynucleotide sequence encoding hSPHK1;
FIG. 5 is a schematic diagram illustrating pUC57_Pgpd_x3myc_Ura_hDES1;
FIG. 6 is a schematic diagram illustrating pUC57_Pgpd_x3myc_Ura_Acer1;
FIG. 7 is a schematic diagram illustrating pUC57_Pgpd_x3myc_Ura_SMase;
FIG. 8 is a schematic diagram illustrating pUC57_Pgall_x3myc_Ura_hSpk1;
FIG. 9 is a schematic diagram illustrating pUC57_Psuc2_x3myc_Ura_hSpk1;
FIG. 10 shows the production of DHS, sphingosine and sphingosine 1-phosphate of the strain prepared in Example 8;
FIG. 11 shows the production of DHS by the strain prepared in Example 8;
FIG. 12 shows the production of sphingosine by the strain prepared in Example 8;
FIG. 13 shows the production of sphingosine 1-phosphate by the strain prepared in Example 8;
FIG. 14 shows the design of a biosynthetic metabolism pathway of sphingosine and sphingosine 1-phosphate in a *Saccharomyces cerevisiae* strain according to an exemplary embodiment of the present invention;
FIG. 15 is a graph illustrating ¹H NMR measurement of sphingosine;
FIG. 16 is a graph illustrating ¹H NMR measurement of sphingosine 1-phosphate; and
FIG. 17 is a graph illustrating ¹H NMR measurement of a cultured cell of the strain prepared in Example 8.

### [Best Mode]

Hereinafter, the present invention will be described in more detail with reference to the following examples. However, these examples are provided only for illustration of the present invention and should not be construed as limiting the scope of the present invention.

### <Example 1: Design of Sphingosine and Sphingosine 1-Phosphate Production Metabolic Pathway

Metabolic pathways for producing sphingosine and sphingosine-1-phosphate were designed to produce mutant strains for producing sphingosine and sphingosine-1-phosphate in *Saccharomyces cerevisiae.* The details thereof were as follows:

### (1) Design of sphingosine production metabolic pathway

### (1-1) Gene insertion

a. Insertion of hDES1 (human sphingolipid desaturase 1) gene to add a pathway to convert dihydrosphingosine (DHS) into sphingosine or to convert dihydroceramide (DHC) into ceramide, which is a sphingosine precursor.
b. Insertion of an ACER1 (alkaline ceramidase 1) gene to add a pathway to convert ceramide to sphingosine.
c. Optionally, insertion of a SMPD1 (sphingomyelin phosphodiesterase 1) gene to add a pathway to convert IPC (inositol-phosphoceramide), MIPC (mannose-inositol-phosphoceramide), or M(IP)2C (mannose-(inositol-P)₂phosphoceramide) into ceramide, which is a sphingosine precursor.

### (1-2) Gene knockout

Deletion of at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase), that are involved in pathways to degrade DHS or DHC or convert the same into other metabolites.

### (1-3) Optionally, gene overexpression

a. overexpression of at least one gene of TSC3LCB1 (serine palmitoyltransferase 1), LCB2 (serine palmitoyltransferase 2), and TSC3 (serine palmitoyltransferase) in order to enhance the reaction of converting palmitoyl-CoA into 3-ketodihydrosphingosine,
b. overexpression of a TSC10 (3-ketodihydrosphingosine reductase) gene to enhance the reaction of converting 3-ketodihydrosphingosine into DHS
c. Overexpression of at least one gene of LAG1 (sphingosine N-acyltransferase), LAC1 (sphingosine N-acyltransferase), and LIP1 (ceramide synthase subunit) to enhance the reaction of converting DHS into DHC

### (2) Design of sphingosine 1-phosphate production metabolic pathway

### (2-1) Gene insertion

a. Insertion of hDES1 (human sphingolipid desaturase 1) gene to add a pathway to convert dihydrosphingosine (DHS) into sphingosine or convert dihydroceramide (DHC) into ceramide, which is a sphingosine precursor.
b. Optionally, insertion of an ACER1 (alkaline ceramidase 1) gene to add a pathway to convert ceramide into sphingosine.
c. Optionally, insertion of an SMPD1 (sphingomyelin phosphodiesterase 1) gene to add a pathway to convert IPC (inositol-phosphoceramide), MIPC (mannose-inositol-phosphoceramide), or M(IP)2C (mannose-(inositol-P)₂phosphoceramide) into ceramide, which is a sphingosine precursor.
d. Insertion of an hSPHK1 (human sphingosine kinase 1) gene to add a pathway that converts sphingosine into sphingosine 1-phosphate

### (2-2) Gene knockout

Deletion of at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase), that are involved in pathways to degrade DHS or DHC or convert the same into other metabolites.

### (2-3) Optionally, gene overexpression

a. overexpression of at least one gene of LCB1 (serine palmitoyltransferase 1), LCB2 (serine palmitoyltransferase 2) and TSC3 (serine palmitoyltransferase) in order to enhance the reaction of converting palmitoyl-CoA into 3-ketodihydrosphingosine,
b. overexpression of a TSC10 (3-ketodihydrosphingosine reductase) gene to enhance the reaction of converting 3-ketodihydrosphingosine into DHS
c. Overexpression of at least one gene of LAG1 (sphingosine N-acyltransferase), LAC1 (sphingosine N-acyltransferase), and LIP1 (ceramide synthase subunit) to enhance the reaction of converting DHS into DHC

### <Example 2: Gene synthesis and expression vector construction>

### (1) Gene synthesis

A gene for insertion into *Saccharomyces cerevisiae* was synthesized.

Specifically, a polynucleotide sequence encoding each of hDES1, hACER1, hSMPD1, and hSPHK1 genes was codon-optimized to ensure excellent expression in *Saccharomyces cerevisiae,* and then each polynucleotide sequence was synthesized. Codon-optimized polynucleotide sequences encoding hDES1, hACER1, hSMPD1 and hSPHK1 are represented by SEQ ID NO: 1, SEQ ID NO: 2, SEQ ID NO: 3, and SEQ ID NO: 4, respectively.

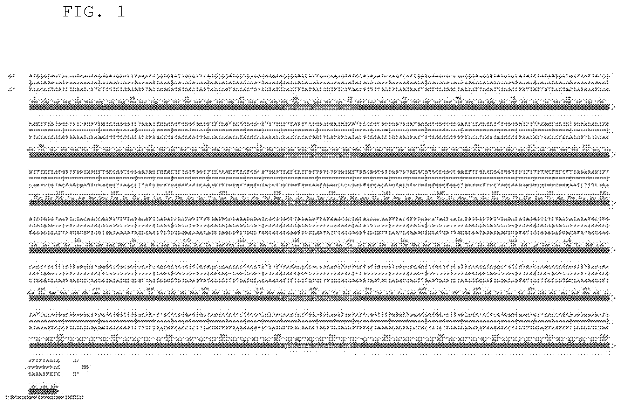

FIG. 1 is a codon-optimized polynucleotide sequence encoding hDES1.

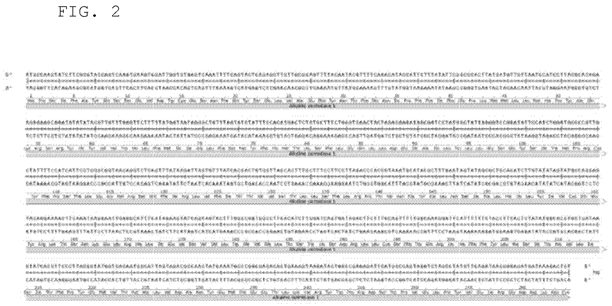

FIG. 2 is a codon-optimized polynucleotide sequence encoding hACER1.

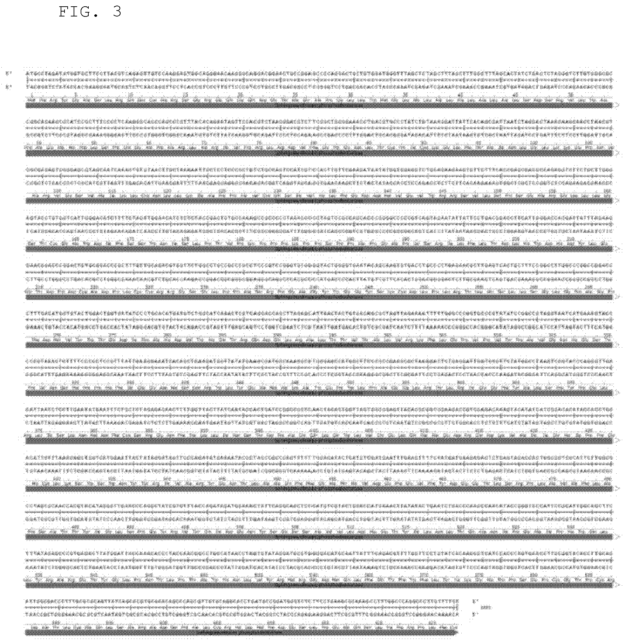

FIG. 3 is a codon-optimized polynucleotide sequence encoding hSMPD1.

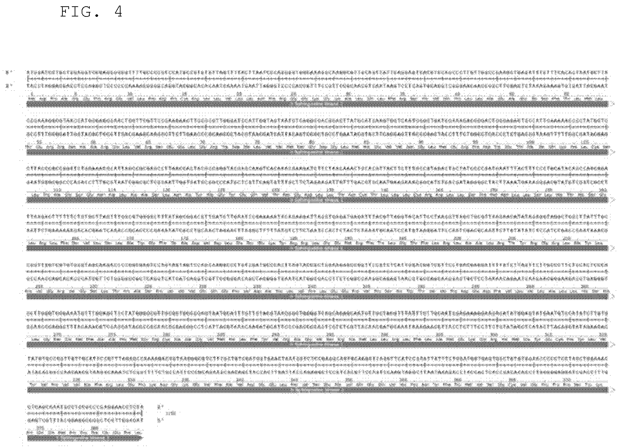

FIG. 4 is a codon-optimized polynucleotide sequence encoding hSPHK1.

### (2) Construction of expression vector

An expression vector was constructed to express the synthesized hDES1, hACER1, hSMPD1 and hSPHK1 polynucleotide sequences in *Saccharomyces cerevisiae.*

Specifically, pUC57_Pgpd_x3myc_Ura was prepared as a basic vector. Each of the synthesized hDES1, hACER1, hSMPD1 and hSPHK1 polynucleotide sequences was ligated to pUC57_Pgpd_x3myc_Ura using restriction enzymes xbaI and xhoI. As a result, the hDES1 expression vector pUC57_Pgpd_x3myc_Ura_hDES1, the hACER1 expression vector pUC57_Pgpd_x3myc_Ura_Acer1, the hSMPD1 expression vector pUC57_Pgpd_x3myc_Ura_SMase, and the hSPHK1 expression vector pUC57_Pgpd: :hSpk1_x3myc_Ura were obtained.

For the hSPHK1 expression vector, to replace the promoter with beta-fructofuranosidase, the SUC2 promoter and the GAL1 promoter were synthesized, were cut using restriction enzymes Sac1 and Xba1, and then were ligated to pUC57_Pgpd: :hSpk1_x3myc_Ura to obtain pUC57_Psuc2: :hSpk1_x3myc_Ura and pUC57_Pgall: :hSpk1_x3myc_Ura, respectively.

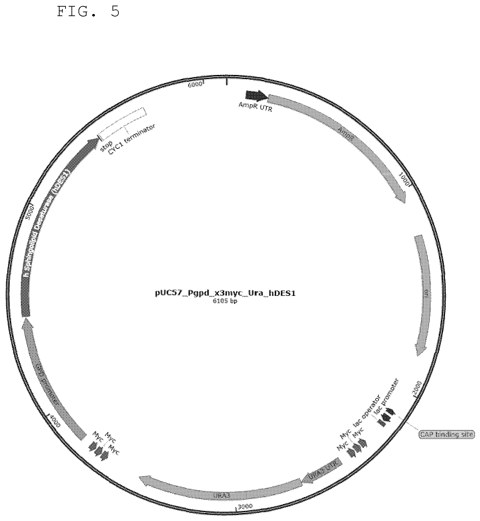

FIG. 5 is a schematic diagram illustrating pUC57_Pgpd_x3myc_Ura_hDES1.

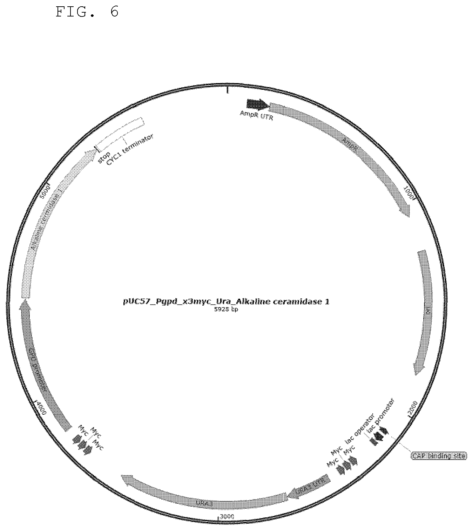

FIG. 6 is a schematic diagram illustrating pUC57_Pgpd_x3myc_Ura_Acer1.

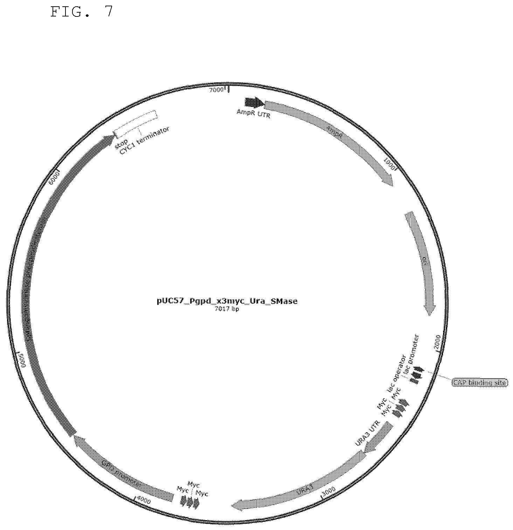

FIG. 7 is a schematic diagram illustrating pUC57_Pgpd_x3myc_Ura_SMase.

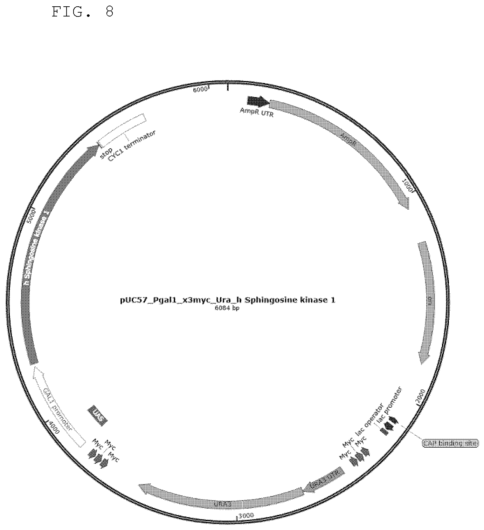

FIG. 8 is a schematic diagram illustrating pUC57_Pgall_x3myc_Ura_hSpk1.

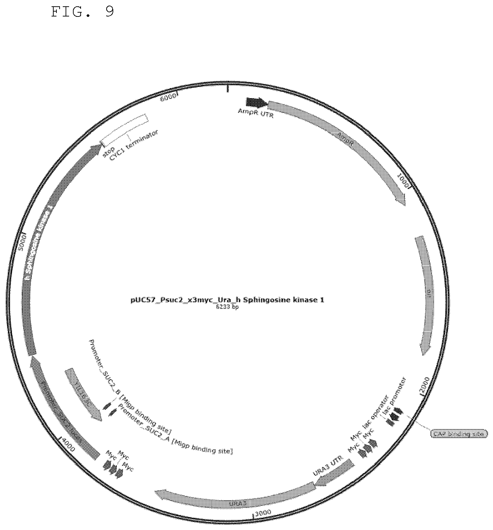

FIG. 9 is a schematic diagram illustrating pUC57_Psuc2_x3myc_Ura_hSpk1.

The prepared expression vectors were transformed into DH5-α cells using EasyComp^{™} and stored.

### (3) Transformation method

Transformation of *Saccharomyces cerevisiae* was performed as follows. *Saccharomyces cerevisiae* grown in YPD (Dextrose 2%) at an OD₆₀₀ of 0.8 was spun down at 3, 700 rpm for 10 minutes. Then, the cells were resuspended, washed with 20 mL of 100 mM lithium acetate (LiAc), resuspended and washed once more with 10 mL of 100 mM lithium acetate. The cells were fractionated with 1.5 mL of 1M lithium acetate and 15% glycerol in a mixer to prepare competent cells.

The competent cells were spun down in a tube at 13,000 rpm for 30 seconds and then the supernatant was removed therefrom. 260 µL of 50% PEG 3500, 36 µL of 1M lithium acetate, 10 µL of SS-Carrier DNA (10 mg/mL), cassette DNA (2 mg to 3 mg) and 14 µL of water were added thereto, followed by mixing with the cells by vortexing. Then, the tube was incubated in a heat block at 30°C for 30 minutes and at 42°C for 1 hour, and spun down at 13,000 rpm for 30 seconds. The supernatant was removed and was then pipetted gently with 1 mL water. 100 µL of the sample was transferred to a YSC-Ura plate and incubated at 30°C for 2 to 3 days.

### <Example 3: Production of DD strain (ΔDPL1::hDES1)>

A *Saccharomyces cerevisiae* mutant strain into which the hDES1 gene was inserted and from which the DPL1 gene was deleted (ΔDPL1::hDES1) was produced, which is referred to as a "DD strain". The details of the production method thereof are as follows.

The pUC57_Pgpd_x3myc_Ura_hDES1 produced in Example 2 was transformed into *Saccharomyces cerevisiae,* to insert the hDES1 gene into *Saccharomyces cerevisiae.*

Then, during insertion of the hDES1 gene, a portion of the DPL1 gene in the *Saccharomyces cerevisiae* was added to the front and rear of the vector pUC57_Pgpd_x3myc_Ura_hDES1 produced in Example 2 to perform transformation. As a result, a fresh hDES1 gene was inserted into the existing DPL1 part and the existing DPL1 gene was then deleted.

### <Example 4: Production of DDLA strain (ΔDPL1::hDES1 ΔLCB5::ACER1)>

A *Saccharomyces cerevisiae* mutant strain (ΔDPL1::hDES1 ΔLCB5::ACER1) into which hDES1 and hACER1 genes were inserted and from which DPL1 and LCB5 genes were deleted was produced, which is referred to as "DDLA strain". The details of the production method are as follows.

The pUC57_Pgpd_x3myc_Ura_Acer1 produced in Example 2 was transformed into the DD strain produced in Example 3, to insert the hACER1 gene into the strain.

Then, during insertion of the ACER1 gene, a portion of the LCB5 gene in the *Saccharomyces cerevisiae* was added to the front and rear of the vector pUC57_Pgpd_x3myc_Ura_Acer1 produced in Example 2 to perform transformation. As a result, a fresh ACER1 gene was inserted into the existing LCB5 part and the existing LCB5 gene was then deleted.

### <Example 5: Production of DDLASS strain (△DPL1::hDES1 △LCB5 ::ACER1 △SUR2 :: SMPD1) >

A *Saccharomyces cerevisiae* mutant strain into which hDES1, hACER1 and hSMPD1 genes were inserted was produced, which is referred to as "DDLASS strain". The details of the production method are as follows.

The pUC57_Pgpd_x3myc_Ura_SMase was transformed into the DDLA strain produced in Example 3, to insert the hACER1 gene into the strain.

Then, during insertion of the SMPD1 gene, a portion of the SUR2 gene in the *Saccharomyces cerevisiae* was added to the front and rear of the vector produced in Example 2 to perform transformation. As a result, a fresh SMPD1 gene was inserted into the existing SUR2 part and the existing SUR2 gene was then deleted.

### <Example 6: Construction of DDOgS strain (△DPL1: :hDES1 △ORM1::Pgal1_hSPHK1)>

A *Saccharomyces cerevisiae* mutant strain into which hDES1 and hSPHK1 genes were inserted was produced, which is referred to as "DDOsS strain". The details of the production method is as follows.

The pUC57_Pgal1_x3myc_Ura_hSpk1 was transformed into the DD strain produced in Example 3, to insert the hSPHK1 gene into the strain.

Then, during insertion of the hSPHK1 gene, a portion of the ORM1 gene in the *Saccharomyces cerevisiae* was added to the front and rear of the vector produced in Example 2 to perform transformation. As a result, a fresh hSPHK1 gene was inserted into the existing ORM1 part and the existing ORM1 gene was then deleted.

### <Example 7: Construction of DDOsS strain (△DPL1::hDES1 △ORM1::Psuc2_hSPHK1)>

A *Saccharomyces cerevisiae* mutant strain into which hDES1 and hSPHK1 genes were inserted was produced, which is referred to as "DDOsS strain". The details of the production method are as follows.

The pUC57_Psuc2_x3myc_Ura_hSpk1 was transformed into the DD strain produced in Example 3, to insert the hSPHK1 gene into the strain.

Then, during insertion of the hSPHK1 gene, a portion of the ORM1 gene in the *Saccharomyces cerevisiae* was added to the front and rear of the vector produced in Example 2. As a result, a fresh hSPHK1 gene was inserted into the existing ORM1 part and the existing ORM1 gene was then deleted.

### <Example 8: Production of DDLAOgS strain (△DPL1::hDES1 △LCB5::ACER1 △ORM1::Pgal1_hSPHK1)>

A *Saccharomyces cerevisiae* mutant strain into which hDES1, hACER1, and hSPHK1 genes were inserted was produced, which is referred to as "DDLAOgS strain". The details of the production method are as follows.

The pUC57_Pgal1_x3myc_Ura_hSpk1 was transformed into the DDLA strain produced in Example 4, to insert the hSPHK1 gene into the strain.

Then, during insertion of the hSPHK1 gene, a portion of the ORM1 gene in the *Saccharomyces cerevisiae* was added to the front and rear of the vector produced in Example 2 to perform transformation. As a result, a fresh hSPHK1 gene was inserted into the existing ORM1 part and the existing ORM1 gene was then deleted.

### <Example 9: Production of DDOsS strain (△DPL1::hDES1 △LCB5::ACER1 △ORM1::Psuc2_hSPHK1)>

A *Saccharomyces cerevisiae* mutant strain into which hDES1, hACER1, and hSPHK1 genes were inserted was produced, which is referred to as "DDLAOsS strain". The details of the production method are as follows.

The pUC57_Psuc2_x3myc_Ura_hSpk1 was transformed into the DDLA strain constructed in Example 4, to insert the hSPHK1 gene into the strain.

Then, during insertion of the hSPHK1 gene, a portion of the ORM1 gene in the *Saccharomyces cerevisiae* was added to the front and rear of the vector produced in Example 2. As a result, a fresh hSPHK1 gene was inserted into the existing ORM1 part and the existing ORM1 gene was then deleted.

### <Experimental Example 1: Culture of strains>

3 mL of YPD (dextrose 2%) was added to a 15 mL round culture tube, and 1 colony of each strain prepared in Example above was added to each tube, followed by pre-culturing overnight. 1 mL of the pre-cultured strain was inoculated into a 500 mL baffle flask containing 100 mL of YPD (dextrose 2%), followed by culturing for 24 hours.

Then, the strain was inoculated at 1% into a 10 L fermenter containing 4L YPD (6% Dextrose), followed by culturing for 144 hours. The strain was fermented in a fed-batch mode at an agitation rate of 200 rpm and at an aeration rate of 0 vvm in the presence of glucose of 5 g/h at a pH of 6. 24 hours after fermentation was started, pH shock was applied using 2M NaOH and 1M HCl as pH solutions along with glucose feeding.

Batch-type fermentation was performed under the same conditions except that glucose feeding was not performed.

### <Experimental Example 2: Evaluation of production of sphingosine and sphingosine 1-phosphate>

### (1) Extraction of sphingolipids

Sphingolipids were extracted from the strain culture solution cultured in Experimental Example 1.

Sphingolipids were extracted by two methods, namely, strong alkaline hydrolysis and bead beater Prep^{™}.

The strong alkaline hydrolysis was performed as follows. A solution of Ba(OH)₂ and 5 mL dioxane in 5 mL 20% water was added to a 200 to 600 mg cell pellet and heated at 110°C for 24 hours. Then, the layers were separated with chloroform/1,4-dioxane/water (8/3/8, v/v/v) and allowed to stand for 15 minutes. The organic layer was obtained and 5 mL of 0.1M KOH and 5 mL of 0.5M KCl were added thereto, followed by allowing to stand for 15 minutes and washing.

The bead beater Prep^{™} was operated as follows. 250 µL of MeOH and 500 µL of CHCl₃ were added to 200 µL of distilled water, and 9 cycles of tissue disruption were performed using a bead beater and a Quickprep^{™} adapter at a rate of 6.0 m/sec for a period of time of 40 seconds. Then, the result was spun down at 1,900 x g for 3 minutes and the lower layer was obtained.

### (2) Preparation of DNP derivatives

5 µL of 1-fluoro-2,4-dinitrobenzene and 4 mL of 2M potassium borate buffer (pH 10.5) were added to the extracted sample, followed by mixing. The mixture was heated at 60°C for 30 minutes and layers were separated into CHCl₃/MeOH/water (8/4/3, v/v/v) to prepare a sphingolipid-DNP (dinitrophenyl) derivative.

### (3) HPLC analysis

A Waters e2695 Module 2998 VWD HPLC system coupled with an autosampler and a UV detector set at 350 nm was used for analysis. The column used herein was an ODS Hypersil RP-C18 (Thermo Electron, 4.6 x 250 mm, 5 um). The flow rate was set to 1 mL/min. Water (A) and methanol-acetonitrile-isopropanol (10/3/1, v/v/v) (B) were used as mobile phases with a linear gradient from 80% B to 90% B over 40 minutes. The equilibration time after the run was 10 minutes and the injection volume was 20 µL.

### (4) Evaluation of production of sphingosine and sphingosine 1-phosphate

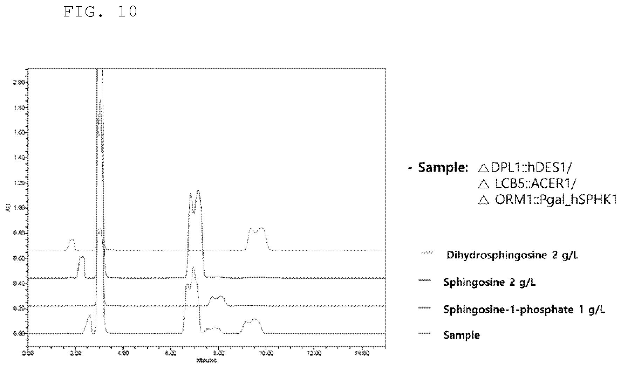

FIG. 10 shows the production of DHS, sphingosine and sphingosine 1-phosphate by the strain prepared in Example 8.

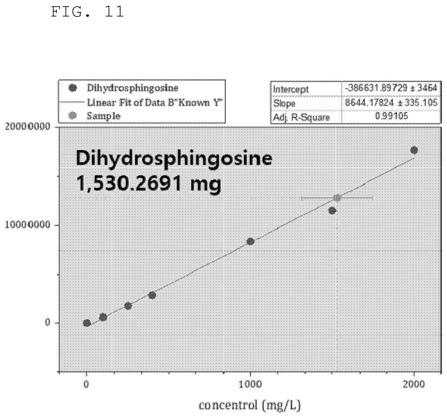

FIG. 11 shows the production of DHS by the strain prepared in Example 8.

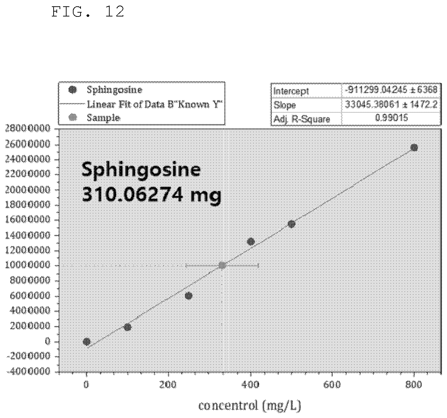

FIG. 12 shows the production of sphingosine by the strain prepared in Example 8.

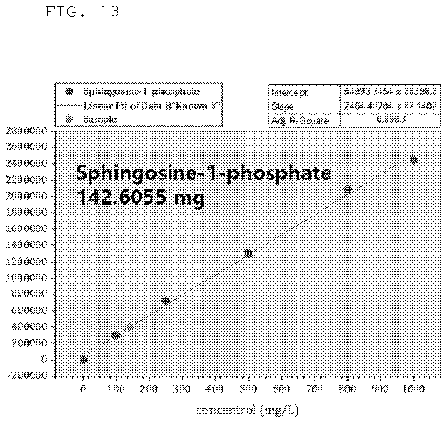

FIG. 13 shows the production of sphingosine 1-phosphate by the strain prepared in Example 8.

As a result, DHS, sphingosine, and sphingosine 1-phosphate production by the DDLAOgS strain were 1530.3 mg/L, 331.1 mg/L, and 142.6 mg/L, respectively.

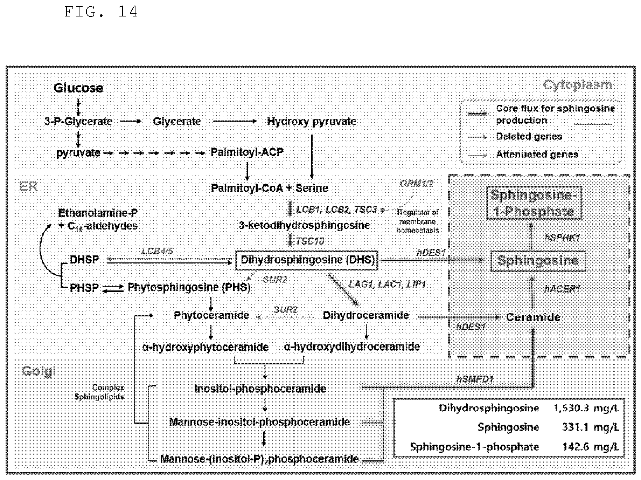

FIG. 14 shows a predicted production pathway of sphingosine and sphingosine 1-phosphate in a *Saccharomyces cerevisiae* strain according to an exemplary embodiment of the present invention. In this embodiment, it is considered that sphingosine and sphingosine 1-phosphate are produced in accordance with the pathway shown in FIG. 14, but the claimed invention is not limited to a specific mechanism.

### <Experimental Example 3: Confirmation of production of sphingosine and sphingosine 1-phosphate>

The strain was pre-cultured at a working volume of 3L in a 5L fermentor for 48 hours and the pre-cultured cells were fermented at a working volume of 3L in a 10L fermentor. The fermentation was started with a starting medium containing 1% yeast extract, 2% peptone, and 6% glucose. After 24 hours, strains using the promoter GAL1 were fed 300 ml/day of 60% galactose and strains using the promoter SUC2 were fed 300 ml/day of 60% sucrose.

Sampling of the fermented product was performed by collecting the product every 12 hours, followed by freeze-drying and extracting sphingolipids on a basis of 1 g of the dried cells.

2 g of glass beads and 5 ml of MeOH were added to 1 g of the dried cells and the cell walls were then disrupted using a bead beater. After disruption of the cell walls, fractionation was performed using 5 ml of chloroform and 1 ml of 0.78% NaCl. After fractionation, the cells were centrifuged at 1,900 x g for 15 minutes at 4°C to collect cell residues to extract the solvent layer in the lower phase.

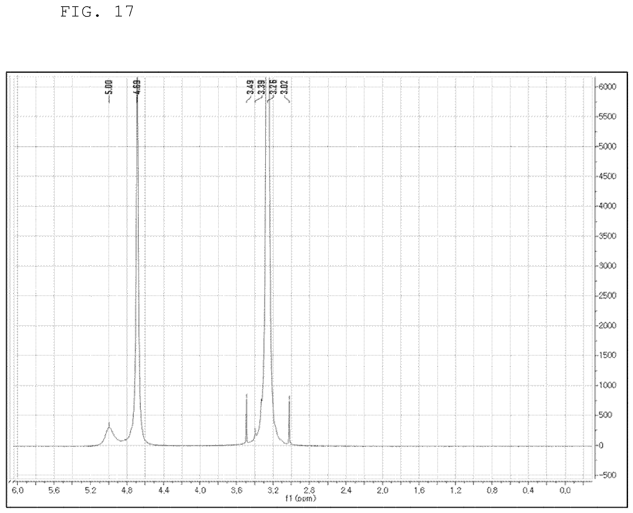

The solvent was distilled off from the extracted solvent layer in a vacuum concentrator at 60°C, and the dried sample was dissolved in a bicarbonate ion solvent and was then subjected to NMR. The results are shown in FIG. 17.

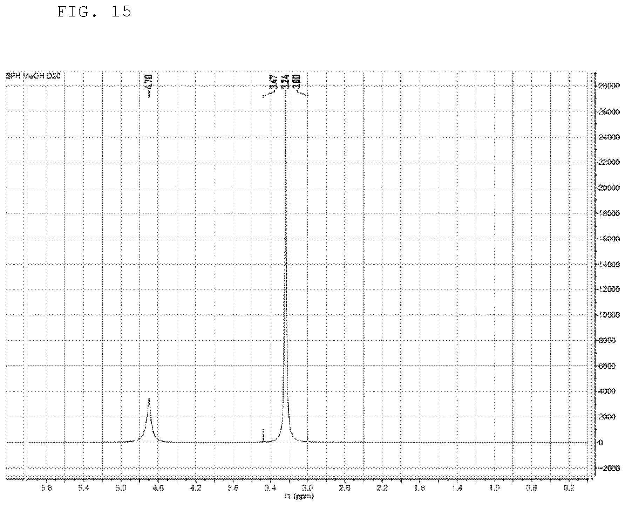

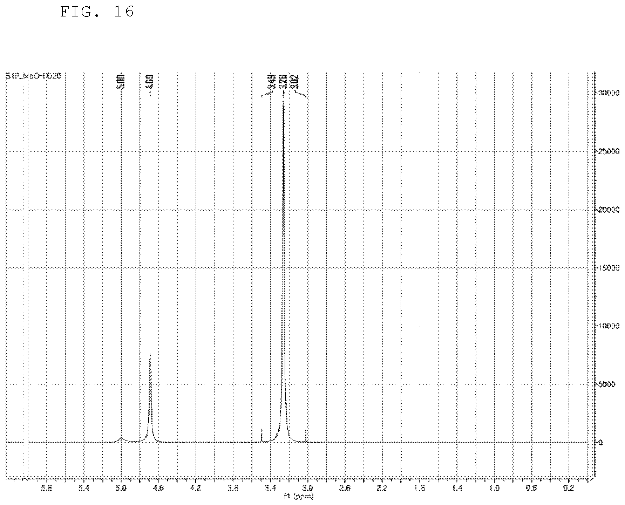

Referring to FIGS. 15 to 17, the peaks observed at about 3.00 to 3.47 eV appeared in both sphingosine and sphingosine 1-phosphate, which indicated the hydrogen of the chain part of the carbon chain, and the peaks observed at approximately -4.7 appeared in both sphingosine and sphingosine 1-phosphate, which indicated the hydrogen at the end of the carbon chain.

In addition, the peak observed at -5.00 was uniquely observed in sphingosine 1-phosphate, which indicated the hydrogen of the phosphate group.

Although embodiments of the present invention have been described in detail, they are merely provided for illustration of the present invention and should not be construed as limiting the scope of the present invention. Those skilled in the art will appreciate that various modifications and applications are possible, without departing from the scope and spirit of the embodiments of the present invention.

Therefore, it should be understood that the scope of the present invention includes alterations, equivalents, or substitutes of the technical ideas exemplified above. For example, each element specifically disclosed in the embodiment of the present invention may be implemented in modified forms. Also, differences related to such modifications and applications should be construed as falling within the scope of the present invention defined in the appended claims.
**SEQ ID NO: 1: hDES1 codon-optimized nucleotide sequence** 
ATGGGCAGTAGAGTCAGTAGAGAAGACTTTGAATGGGTCTATACGGATCAGCCGCATG

CTGACAGGAGAAGGGAAATATTGGCAAAGTATCCAGAAATCAAGTCATTGATGAAGCC

CGACCCTAACCTAATCTGGATAATAATAATGATGGTACTTACCCAACTTGGTGCATTT

TACATTGTAAAGGATCTAGATTGGAAGTGGGTAATCTTTGGTGCATACGCCTTTGGGT

CATGTATCAACCACAGTATGACCCTAGCGATTCATGAAATCGCCCACAACGCAGCATT

TGGGAATTGTAAGGCGATGTGGAACAGGTGGTTTGGCATGTTTGCTAACTTGCCAATC

GGAATACCGTACTCTATTAGTTTCAAACGTTATCACATGGATCACCATCGTTATCTGG

GGGCTGACGGTGTTGATGTAGACATACCGACCGACTTCGAAGGATGGTTCTTCTGTAC

TGCCTTTAGAAAGTTTATCTGGGTGATTCTGCAACCACTATTTTATGCGTTCAGACCG

CTGTTTATAAATCCCAAACCGATCACATACTTAGAGGTTATAAACACTGTAGCGCAAG
 
TTACTTTTGACATACTAATCTATTATTTTTTGGGCATAAAGTCTCTAGTGTATATGCT

TGCAGCTTCTTTATTGGGGTTGGGTCTGCACCCAATCAGCGGACACTTCATAGCCGAA

CACTACATGTTTTTAAAAGGACACGAAACGTACTCTTATTATGGTCCGCTGAATTTAC

TTACATTCAACGTAGGCTATCATAACGAACACCACGATTTTCCGAATATCCCAGGGAA

GAGCCTTCCACTGGTTAGAAAAATTGCAGCGGAGTACTACGATAATCTTCCACATTAC

AACTCTTGGATCAAGGTTCTATACGATTTTGTGATGGACGATACAATTAGCCCATACT

CCAGGATGAAACGTCACCAGAAGGGGGAGATGGTTTTAGAG
**SEQ ID NO: 2: ACER1 codon-optimized nucleotide sequence** 
ATGCCAAGTATCTTCGCGTACCAGTCAAGTGAAGTGGATTGGTGTGAGTCAAATTTTC

AGTACTCAGAGCTTGTTGCGGAGTTTTACAATACGTTTTCAAACATACCATTCTTTAT

ATTCGGCCCACTTATGATGTTGTTAATGCATCCTTACGCACAGAAGAGAAGCAGATAT

ATATACGTTGTTTGGGTTCTTTTTATGATAATAGGGCTGTTTAGTATGTATTTCCACA

TGACTCTGTCCTTTCTGGGTCAACTACTAGACGAGATAGCGATCCTATGGCTATTAGG

GTCCGGATATTCCATCTGGATGCCCCGTTGCTATTTTCCATCATTCCTGGGCGGTAAC

AGGTCTCAGTTTATAAGATTAGTGTTTATCACGACTGTGGTTAGCACTTTGCTTTCCT

TCCTTAGACCCACCGTAAATGCATACGCTTTGAATAGTATAGCGCTGCACATCTTGTA

TATAGTATGCCAGGAGTACAGGAAAACTTCAAATAAGGAATTGAGGCATTTGATAGAA

GTATCAGTAGTACTTTGGGCGGTGGCCTTGACATCTTGGATCAGTGATAGACTTCTTT

GCAGTTTTTGGCAAAGGATTCATTTTTTCTACCTTCACTCTATATGGCACGTGCTGAT

AAGTATCACGTTCCCTTACGGTATGGTGACAATGGCATTAGTAGACGCCAACTATGAA

ATGCCCGGCGAGACACTGAAAGTAAGATACTGGCCGAGAGATTCATGGCCAGTAGGTC

TACCCTATGTTGAGATAAGGGGAGATGATAAAGACTGT
**SEQ ID NO: 3: SMPD1 codon-optimized nucleotide sequence** 
ATGCCTAGATATGGTGCTTCCTTACGTCAGAGTTGTCCAAGGAGTGGCAGGGAACAAG

GGCAGGACGGAACTGCCGGAGCCCCAGGACTGCTGTGGATGGGTTTAGCTCTAGCTTT

AGCTTTGGCTTTAGCACTATCTGACTCTAGGGTCTTGTGGGCGCCGGCAGAAGCGCAT

CCGCTTTCCCCTCAAGGGCACCCAGCCCGTTTACACAGAATAGTTCCACGTCTAAGGG

ACGTCTTCGGCTGGGGAAACCTGACGTGCCCTATCTGTAAAGGATTATTCACAGCGAT

TAATCTAGGACTAAAGAAGGAACCTAACGTGGCGAGAGTGGGGAGCGTAGCAATCAAA

CTGTGTAACTTGCTAAAAATTGCTCCTCCCGCTGTCTGCCAGTCCATCGTCCACTTGT

TCGAAGATGATATGGTGGAGGTCTGGAGAAGAAGTGTTCTTTCACCGAGCGAGGCCAG

AGGTCTTCTGCTTGGGAGTACCTGTGGTCATTGGGACATCTTTTCTAGTTGGAACATC

TCTCTACCGACTGTGCCAAAGCCGCCCCCTAAACCCCCTAGTCCGCCAGCACCCGGGG

CCCCCGTCAGTAGAATATTATTCCTGACGGACCTTCATTGGGACCACGATTATTTAGA

AGGAACGGACCCGGACTGTGCGGACCCGCTTTGTTGCAGACGTGGTTCTGGCCTGCCG

CCCGCCTCCCGTCCGGGTGCGGGGTACTGGGGTGAATACAGCAAGTGTGACCTGCCCC

TGAGAACGCTTGAGTCACTGCTTTCCGGCCTTGGCCCGGCCGGACCCTTTGACATGGT

GTACTGGACTGGTGATATCCCTGCACATGATGTCTGGCATCAAACTCGTCAGGACCAG

CTTAGAGCATTAACTACTGTGACAGCGCTAGTTAGAAAATTTTTGGGCCCGGTGCCCG

TATATCCGGCCGTAGGTAATCATGAAAGTACCCCCGTAAACTCTTTTCCCCCTCCGTT

TATTGAAGGAAATCACAGCTCAAGATGGTTATATGAAGCGATGGCAAAGGCGTGGGAG

CCATGGCTTCCCGCCGAAGCGCTAAGGACTCTGAGGATTGGTGGGTTCTATGCCCTAA

GTCCGTACCCAGGGTTGAGATTAATCTCCTTGAATATGAATTTCTGCTCTAGAGAGAA

CTTTTGGTTACTTATCAATAGCACTGATCCGGCCGGTCAACTACAGTGGTTAGTGGGG

GAGTTACAGGCGGCGGAAGACCGTGGAGACAAAGTACATATCATCGGACACATACCAC

CTGGACATTGTTTAAAGAGCTGGTCATGGAATTACTATAGGATAGTTGCCAGATATGA

AAATACGCTAGCCGCCCAGTTTTTTGGACATACTCATGTCGATGAATTTGAAGTTTTC

TATGATGAAGAGACTCTGAGTAGACCACTGGCGGTCGCATTCTTGGCGCCTAGCGCAA
 
CCACGTACATAGGGTTGAACCCAGGCTATCGTGTTTACCAGATAGATGGAAACTATTC

AGGCAGCTCCCATGTCGTACTGGACCATGAAACTTATATACTGAATCTGACCCAAGCC

AACATACCGGGTGCCATTCCGCATTGGCAGCTTCTTTATAGAGCCCGTGAGACTTATG

GATTACCAAACACCCTACCAACGGCCTGGCATAACCTAGTGTATAGGATGCGTGGGGA

CATGCAATTATTTCAGACGTTTTGGTTCCTGTATCACAAGGGTCATCCACCCAGTGAA

CCTTGCGGTACACCTTGCAGATTGGCGACCCTTTGCGCGCAGTTATCAGCGCGTGCGG

ACAGCCCAGCGTTGTGCAGGCACCTGATGCCGGATGGGTCTCTTCCTGAAGCGCAAAG

CCTTTGGCCCAGGCCCTTGTTTTGT
**SEQ ID NO: 4: hSPHK1 codon-optimized nucleotide sequence**

ATGGATCCTGCTGGAGGTCCGAGGGGGGTTTTGCCCCGTCCATGCCGTGTGTTAGTTT

TACTTAATCCCAGGGGTGGCAAAGGCAAGGCGTTGCAGTTATTCAGGAGTCACGTCCA

GCCCTTGTTGGCCGAAGCTGAGATTTCTTTCACACTAATGCTTACGGAAAGGCGTAAC

CATGCGAGGGAACTGGTTCGTTCCGAAGAACTTGGGCGTTGGGATGCATTGGTAGTAA

TGTCAGGCGACGGACTTATGCATGAAGTGGTCAATGGGCTGATGGAAAGACCGGACTG

GGAGACTGCCATTCAAAAACCGCTATGCTCCTTACCCGCCGGGTCTGGAAACGCATTA

GCCGCGAGCCTTAACCACTACGCCGGGTACGAGCAAGTCACAAACGAAGATCTTCTAA

CAAACTGCACGTTACTTCTTTGCCGTAGACTGCTATCCCCGATGAATTTACTTTCCCT

GCATACAGCCAGCGGATTAAGACTTTTTTCTGTGCTTAGTTTGGCGTGGGGCTTTATA

GCGGACGTTGATCTTGAATCCGAAAAATACAGAAGATTAGGTGAGATGAGATTTACGT

TAGGTACATTCCTAAGGTTAGCTGCGTTAAGAACATATAGAGGTAGGCTGGCTTATTT

GCCGGTTGGTCGTGTGGGTAGCAAGACCCCGGCGAGTCCAGTAGTAGTCCAGCAAGGC

CCTGTCGATGCCCACTTAGTACCGCTGGAAGAGCCGGTTCCGTCTCATTGGACGGTCG

TTCCTGACGAGGATTTTGTCTTAGTTCTTGCCCTTCTGCACTCCCACCTTGGGTCCGA

AATGTTTGCAGCTCCTATGGGGCGTTGCGCTGCCGGAGTAATGCATTTGTTCTACGTA

AGGGCTGGGGTCAGCAGAGCAATGTTGCTACGTTTATTTCTTGCAATGGAGAAAGGAA

GACATATGGAGTATGAATGTCCATATCTTGTGTATGTGCCGGTTGTTGCATTCCGTTT

AGAGCCAAAAGACGGTAAGGGCGTCTTCGCTGTCGATGGTGAACTAATGGTCTCCGAG

GCAGTGCAAGGTCAGGTTCATCCGAATTATTTCTGGATGGTGAGTGGCTGTGTGGAAC

CCCCTCCTAGCTGGAAACCTCAGCAAATGCCTCCGCCCGAGGAACCTCTA

## Claims

1. A *Saccharomyces cerevisiae* mutant strain for producing sphingosine into which a hDES1 (human sphingolipid desaturase 1) gene and an ACER1 (alkaline ceramidase 1) gene are introduced.

2. The *Saccharomyces cerevisiae* mutant strain according to claim 1, wherein a SMPD1 (sphingomyelin phosphodiesterase 1) gene is further introduced into the strain.

3. The *Saccharomyces cerevisiae* mutant strain according to claim 1, wherein the strain has reduced activity of at least one enzyme involved in a pathway for degrading DHS (dihydrosphingosine) or converting the DHS into another product.

4. The *Saccharomyces cerevisiae* mutant strain according to claim 1, wherein the strain has reduced activity of at least one enzyme involved in a pathway for degrading DHC (dihydroceramide) or converting the DHC into another product.

5. The *Saccharomyces cerevisiae* mutant strain according to claim 1, wherein at least one of LCB4 (sphingoid long chain base kinase 4), LCB5 (sphingoid long chain base kinase 5), DPL1 (dihydrosphingosine phosphate lyase), and SUR2 (sphinganine C4-hydroxylase) is deleted or disrupted.

6. The *Saccharomyces cerevisiae* mutant strain according to claim 1, wherein the strain has improved activity of an enzyme that catalyzes a reaction of converting palmitoyl-CoA into 3-ketodihydrosphingosine.

7. The *Saccharomyces cerevisiae* mutant strain according to claim 6, wherein the strain overexpresses at least one of serine palmitoyltransferase 1 (LCB1), serine palmitoyltransferase 2 (LCB2), and serine palmitoyltransferase-regulating protein (TSC3).

8. The *Saccharomyces cerevisiae* mutant strain according to claim 1, wherein the strain has improved activity of an enzyme that catalyzes a reaction of converting 3-ketodihydrosphingosine into DHS.

9. The *Saccharomyces cerevisiae* mutant strain according to claim 8, wherein the strain overexpresses a TSC10 (3-ketodihydrosphingosine reductase) gene.

10. The *Saccharomyces cerevisiae* mutant strain according to claim 1, wherein the strain has improved activity of an enzyme that catalyzes a reaction of converting DHS into DHC.

11. The *Saccharomyces cerevisiae* mutant strain according to claim 10, wherein the strain overexpresses at least one of LAG1 (sphingosine N-acyltransferase), LAC1 (sphingosine N-acyltransferase), and LIP1 (ceramide synthase subunit).

12. A *Saccharomyces cerevisiae* mutant strain for producing sphingosine 1-phosphate, constructed by further introducing a hSPHK1 (human sphingosine kinase 1) gene into the *Saccharomyces cerevisiae* mutant strain according to any one of claim 1 to 11.

13. A method of producing a *Saccharomyces cerevisiae* mutant strain for producing sphingosine, the method comprising:
introducing a hDES1 (human sphingolipid desaturase 1) gene and an ACER1 (alkaline ceramidase 1) gene into a *Saccharomyces cerevisiae* strain.

14. The method according to claim 13, further comprising at least one of the following steps:
introducing a SMPD1 (sphingomyelin phosphodiesterase 1) gene into the *Saccharomyces cerevisiae* strain;
deleting at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase) from the *Saccharomyces cerevisiae* strain; and
overexpressing at least one gene of LCB1 (serine palmitoyltransferase 1), LCB2 (serine palmitoyltransferase 2), TSC3 (serine palmitoyltransferase-regulating protein TSC3), TSC10 (3-ketodihydrosphingosine reductase), LAG1 (sphingosine N-acyltransferase), LAC1 (sphingosine N-acyltransferase), and LIP1 (ceramide synthase subunit) in the *Saccharomyces cerevisiae.*

15. A method of producing a *Saccharomyces cerevisiae* mutant strain for producing sphingosine 1-phosphate, the method comprising:
introducing a hDES1 (human sphingolipid desaturase 1) gene, an ACER1 (alkaline ceramidase 1) gene, and a hSPHK1 (human sphingosine kinase 1) gene into a *Saccharomyces cerevisiae* strain.

16. The method according to claim 15, further comprising at least one of the following steps:
introducing a SMPD1 (sphingomyelin phosphodiesterase 1) gene into the *Saccharomyces cerevisiae* strain;
deleting at least one gene of LCB4 (sphingoid long chain base kinase 4), LCB5 (sphingoid long chain base kinase 5), DPL1 (dihydrosphingosine phosphate lyase), and SUR2 (sphinganine C4-hydroxylase) from the *Saccharomyces cerevisiae* strain; and
overexpressing at least one gene of LCB1 (serine palmitoyltransferase 1), LCB2 (serine palmitoyltransferase 2), TSC3 (serine palmitoyltransferase-regulating protein TSC3), TSC10 (3-ketodihydrosphingosine reductase), LAG1 (sphingosine N-acyltransferase), LAC1 (sphingosine N-acyltransferase), and LIP1 (ceramide synthase subunit) in the *Saccharomyces cerevisiae.*

17. A method of producing sphingosine comprising culturing the *Saccharomyces cerevisiae* mutant strain according to claim 11 in a medium.

18. The method according to claim 17, further comprising collecting the sphingosine from a culture product.

19. A method of producing sphingosine 1-phosphate comprising culturing the strain according to claim 12 in a medium.

20. The method according to claim 19, further comprising collecting the sphingosine 1-phosphate from a culture product.
